# EUROPEAN PATENT SPECIFICATION

(11) **EP 0 999 808 B1**
(45) Date of publication and mention of the grant of the patent: **29.12.2004**
(21) Application number: 98938198.3
(22) Date of filing: 30.07.1998
(51) Int. Cl.: A61F 2/44

(54) **CERVICAL DISK AND SPINAL STABILIZER**
ZERVIKALE-BANDSCHEIBE UND RÜCKGRAT-STABILISIERUNG
DISQUE CERVICAL ET STABILISATEUR VERTEBRAL

(30) Priority: 01.08.1997 US 904856
(43) Date of publication of application: 17.05.2000
(73) Proprietor: Perumala Corporation, Brownsville, TX 78520-8817 (US)
(72) Inventor: PISHARODI, Madhavan, Houston, TX 78520-8817 (US)
(74) Representative: Frankland, Nigel Howard
(86) International application number: PCT/US1998/015940
(87) International publication number: WO 1999/005994

(56) References cited:
- WO-A-97/46165
- GB-A- 1 243 353
- US-A- 4 401 112
- US-A- 4 599 086

## Description

The present invention relates to an intervertebral disk stabilizing implant for use in the cervical region of the spinal column and a method of stabilizing two adjacent cervical vertebrae. More specifically, the present invention relates to rectangularly-shaped disk implants which are expanded in the middle portion and are used for fusion of the cervical spine.

Treatment of a herniated disk in the neck and in the lumbar region continues to be a challenging field of medicine. The classical treatment for a ruptured disk is diskectomy, i.e., removal of the disk from between the vertebrae. In this process, all or a portion of the intervertebral disk is removed, leaving a defect which may continue to bother the patients throughout the rest of their lives. An additional procedure is to replace the disk space with a bone graft, usually bone chips cut from the patient's iliac crest, bringing about fusion of the vertebrae above and below the disk, eliminating the empty space between the vertebrae.

No prosthetic disk has been shown to be safe and efficacious to date, so the best alternative treatment currently available is a to fuse the adjacent vertebrae. However, diskectomy with fusion is not ideal because the replaced bone does not have the function of the cartilaginous tissue of the disk, i.e. no cushioning effect, and has complications because of several factors. First, conventional bone plugs used to pack the disk space do not conform to the space of the disk because the disk bulges in the center. The disk space is wider in the middle and narrower at its anterior and posterior ends. For this reason, the various currently available commercial bone plugs have only four contact points, i.e. at the front and back of the disk space, such that they are not as stable as is desirable. Second, access to the disk is from the side of the dorsal spine of the adjacent vertebrae, leaving a space that is "off-center" relative to the bodies of the vertebrae such that the stability of the implant is even more problematical than might be apparent from the limited contact resulting from the shape of the disk space. Another complication is the possibility of infection or other conditions which may require the removal of the implant. Also, if the bone pieces do not fuse, they may eventually extrude out of the disk space, causing pressure on the nerve roots.

Prosthetic disk plugs, or implants, are disclosed in the art, but all are characterized by limitations of not conforming to the shape of the disk space, instability when inserted off-center, inability to be removed, or other disadvantages. For instance, U.S. Patent No. 4,863,476 (and its European counterpart, EP-A-0260044) describes a longitudinally divided, elongate body with a cam device for increasing the space between the two body portions once inserted into the disk space. However, that device is cylindrical in shape such that the only contact points with the vertebral bodies are at the front and back of the disk space such that this device is characterized by the same instability as other disk plugs. The art also discloses intervertebral disk prostheses (U.S. Patent Nos. 3,867.728, 4.309,777, 4,863,477 and 4,932,969 and French No. 8816184) which may have more general contact with the adjacent disks, but which are not intended for use in fusion of the disks. The art also includes spinal joint prostheses such as is described in U.S. Patent No. 4,759,769, which is again not indicated for use when fusion is the preferred surgical intervention.

The problem of maintaining the spacing between vertebrae is particularly acute in the cervical vertebrae. The surgery itself is not as difficult as in the lumbar spine because access is from the front in the cervical spine, e.g.. ventrally to the patient. Because bone chips are not substantial enough to maintain the spacing between vertebrae, the accepted surgical method to maintain spacing between adjacent vertebrae in the cervical spine is to scoop out the entire damaged disk, drill a hole into the intervertebral space, and insert a plug of the patient's bone into the disk space. However, cervical vertebrae are smaller than the vertebrae in the rest of the spinal column, so there is little tolerance for anterior/posterior movement of the bone plug in the disk space such as may occur before the fusion is complete. Because of the relatively small tolerances, almost any movement posteriorally in the disk space imperils the nerves of the spinal cord, and bone plugs have no structure to resist such movement. Consequently, the hole into which the plug is inserted is slightly undersized relative to the plug so that the ligaments on either side of the spinal column help hold the plug in place by compressing the plug between vertebrae. Even so, the plug can move in the intervertebral space, so there is a need for a spinal implant for stabilizing adjacent cervical vertebrae which maintains sufficient space between the vertebrae to allow the spinal nerves to pass between the processes of the cervical vertebrae without impingement by the vertebrae and which does not allow anterior/posterior movement of the implant in the disk space.

Similarly, the bone chips packed into the disk space around the plug can be extruded posteriorally against the spinal cord or dorsal roots by the compression provided by the ligaments which help hold the bone plug in place. There is, therefore, a need for an implant which protects against impingement of the spinal cord and/or nerves. There is also a need for a cervical implant which can be removed in the event of infection or other post-surgical complication. Likewise, there is a need for an implant for stabilizing more than two vertebrae of the cervical spine without imperiling the spinal cord and nerves and without placing screws into the spinal bodies.

GB-A-1,243,353 discloses a prosthetic appliance in the form of an artificial vertebra to be used to bridge a gap left in a spinal column when one or more of the natural vertebrae have been removed. The device comprises a two-part body that can be mounted around the spinal column, and longitudinally extending brace elements which lie adjacent and are secured to the neural spine of adjacent vertebrae.

US-A-4,599,086 also discloses a prosthesis adapted to replace a vertebra. The prosthesis incorporate a plate 66 which is aligned with the axis of the spine and is fastened to the bony structure of adjacent vertebrae.

WO97/46165 relates primarily to a surgical instrument for the fusion of vertebrae. The device includes a body located between adjacent vertebrae and a protruding plate-like element that lies in contact with the adjacent vertebrae, and which is secured to the adjacent vertebrae by screws.

The present invention seeks to provide a cervical disk stabiliser for insertion between originally adjacent vertebrae.

According to this invention there is provided a cervical disk stabiliser wherein the stabiliser is for insertion between originally adjacent vertebrae, the stabiliser comprising an elongate implant having a substantially rectangular cross-sectional shape for inserting between originally adjacent cervical vertebrae; a stabiliser bar for detachable mounting to one end of said implant and means to prevent relative rotation between the stabiliser bar and the implant when the stabiliser bar is mounted on the implant, there being ears formed on the stabiliser bar for insertion into the disc space between said adjacent vertebrae, said ears having surfaces for bearing against the adjacent cervical vertebrae, to prevent movement of said stabiliser bar relative to the adjacent cervical vertebrae.

Conveniently said protector extends outwardly from said implant at an angle of about 90° relative to the longitudinal axis of said implant.

Advantageously said protector is pivotally mounted to said implant.

Preferably said protector is adapted to pivot from a first, folded position against said implant to a second, extended position extending orthogonally from said implant.

Conveniently said protector is adapted to be pivoted from a first, folded position for inserting said implant into the space between adjacent cervical vertebrae, and second position extending outwardly from said implant at an angle relative to the longitudinal axis of said implant for closing off the dorsal aspect of the space between adjacent cervical vertebrae.

Advantageously the stabiliser additionally comprises means for restraining the pivoting of said protector from the first, folded position past said second, extended position.

Preferably said restraining means is breakable so as to allow said protector to pivot past said second, extended position to a third position approximately 180° from said first, folded position.

Conveniently said protector is provided with a plurality of vertically aligned grooves for allowing said protector to bend.

Advantageously the longitudinal axis of said stabiliser bar is positioned at an angle of about 90° to the longitudinal axis of said implant.

Preferably the stabiliser bar is curved to approximate the shape of ventral aspect of a vertebra.

Conveniently the stabiliser additionally comprises a connector for detachably mounting to said implant when implanted between adjacent cervical vertebrae for connecting the implant to an adjacent implant.

Advantageously the implant is comprised of sections that are movable with respect to each other.

Thus a typical cervical disk stabiliser in accordance with the invention comprises an elongate member having a substantially rectangular cross-sectional shape for inserting between adjacent cervical vertebrae and a stabilizer bar for detachably mounting to one end of the elongate member to prevent relative rotation therebetween. An ear is formed on the stabilizer bar for inserting between the adjacent cervical vertebrae, and because the stabilizer bar is mounted to the elongate member in such a way as to prevent relative rotation, if the elongate member even starts to rotate in the space between adjacent cervical vertebrae, the ear contacts one of the bodies of the adjacent vertebrae to prevent rotation of the elongate member. The stabilizer bar is elongate and the long axis of the stabilizer bar is mounted orthogonally to the longitudinal axis of the elongate member.

Also provided is an implant comprised of an elongate member formed in multiple, interconnected parts which, when assembled, provide the member with a substantially rectangularcross-sectional shape for inserting between adjacent cervical vertebrae. A stabilizer bar is provided for detachably mounting to one end of the elongate member to prevent relative rotation therebetween with an ear formed thereon for inserting between the adjacent cervical vertebrae. Because the stabilizer bar is mounted to the elongate member in such a way as to prevent relative rotation, if the elongate member starts to rotate in the space between adjacent cervical vertebrae, the ear contacts one of the bodies of the adjacent vertebrae to prevent rotation of the elongate member. The stabilizer bar is elongate and the long axis of the stabilizer bar is mounted orthogonally to the longitudinal axis of the elongate member. If the implant must be removed, the stabilizer bar is removed and the parts of the elongate member are dis-assembled in the disk space so that any bone chips which have fused in the disk space are not disturbed upon removal of the elongate member.

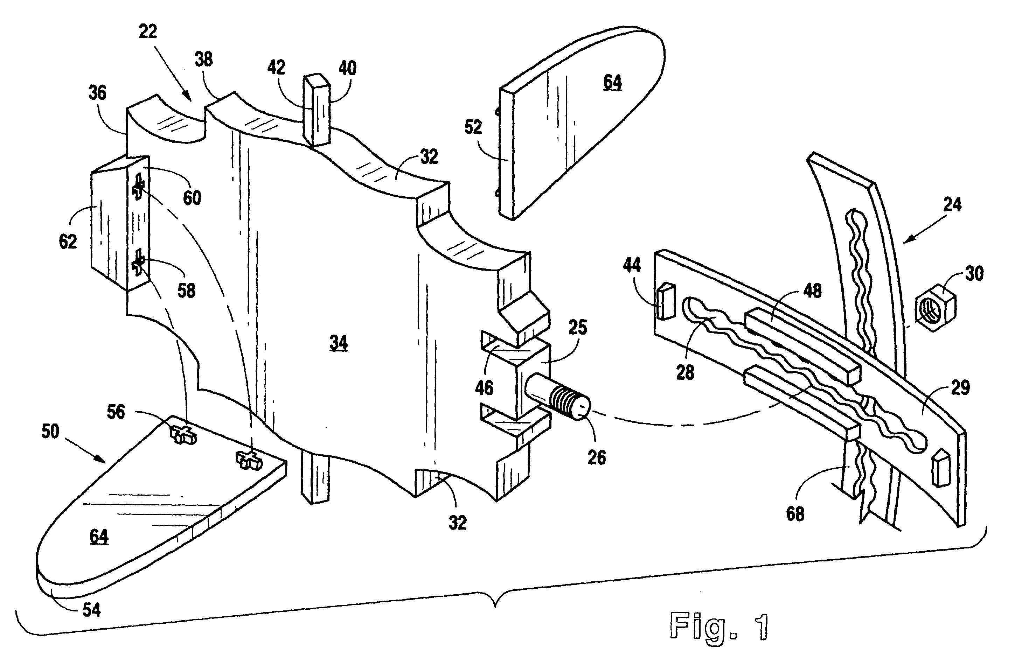

Referring now to the figures, a perspective view of a cervical stabilizer constructed in accordance with the teachings of the present invention is shown in Fig. 1.
Figure 2 is a side view of the human spinal column showing the cervical stabilizer of Fig. 1 inserted in the intervertebral space and having a portion of the adjacent vertebrae cut away to show the manner in which the stabilizer interacts with the adjacent vertebrae.
Figure 3 is a top plan view of a single cervical vertebrae showing the anterior-posterior positioning of the cervical stabilizer of Fig. 1 relative to the body of the vertebrae.
Figure 4 is a schematic, perspective view of the ventral aspect of the vertebral bodies of adjacent cervical vertebrae having the cervical stabilizer of Fig. 1 inserted between each vertebrae.
Figure 5 is a partially schematic view of adjacent cervical vertebrae similar to the view shown in Fig. 4 showing the two cervical stabilizers before connection with a spinal stabilizer constructed in accordance with the teachings of the present invention.
Figure 6 is a partially schematic view of three adjacent cervical vertebrae similar to the view shown in Fig. 4 and having spinal stabilizer connecting the two cervical stabilizers.
Figure 7 is a perspective view of a second embodiment of a cervical stabilizer constructed in accordance with the teachings of the present invention.
Figure 8 is a perspective view of a third embodiment of a cervical stabilizer constructed in accordance with the teachings of the present invention.
Figure 8A is an end elevational view of an alternative embodiment of the cervical stabilizer shown in Fig. 8.
Figure 9 is a perspective view of a fourth embodiment of a cervical stabilizer constructed in accordance with the teachings of the present invention.
Figure 10 is a perspective view of a fifth embodiment of a cervical stabilizer constructed in accordance with the teachings of the present invention.
Figure 11 is a perspective view of a sixth embodiment of a cervical stabilizer constructed in accordance with the teachings of the present invention.
Figure 12 is a perspective view of a seventh embodiment of a cervical stabilizer constructed in accordance with the teachings of the present invention.
Figure 13 is a schematic, perspective view of the ventral aspect of the vertebral bodies of adjacent cervical vertebrae having an eighth embodiment of the cervical stabilizer constructed in accordance with the teachings of the present inserted between the second and third vertebrae. Figure 14 is a side elevational view of an ninth embodiment of a cervical stabilizer constructed in accordance with the teachings of the present invention.
Figure 15 is an end elevational view of the cervical stabilizer of Fig. 14.
Figure 16 is a side view of a tenth embodiment of the cervical stabilizer of the present invention having a portion of the adjacent vertebrae cut away to show the manner in which portions of the implant comprising the stabilizer interacts with each other.
Figure 17 is a side view of the human spinal column showing an eleventh embodiment of the cervical stabilizer of the present invention inserted in the intervertebral space and having a portion of the adjacent vertebrae cut away to show the manner in which the stabilizer interacts with the adjacent vertebrae.
Figure 18 is a top plan view of a twelfth embodiment of the cervical stabilizer of the present invention.

Referring now to the cervical stabilizer shown in Fig. 1, the stabilizer is comprised of an elongate implant 22, a lock indicated generally at reference numeral 24, and means for detachably mounting the lock to one end 25 of implant 22. In the embodiment shown, the mounting means takes the form of a bolt, or threaded post, 26 passing through a bore 28 in the stabilizer bar 29 comprising lock 24, the threads of post 26 engaging threads in the nut 30 on the end of post 26.

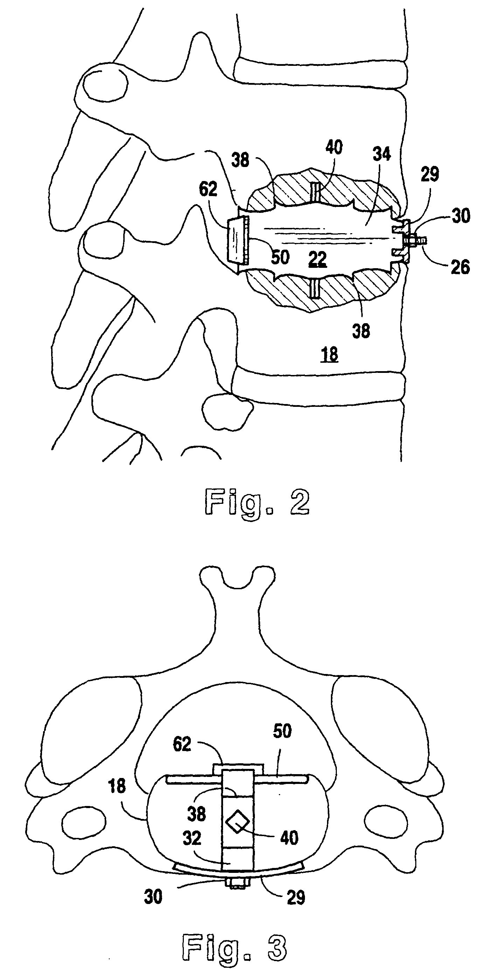

Implant 22 is an elongate member comprised of first and second sides 32 and third and fourth sides 34 providing a substantially rectangular-shaped cross-sectional shape. The height H of the rectangularly-shaped cross-section is defined by sides 32 and the width W is defined by sides 34. As is apparent by comparison of H to W, the height H of implant 22 is less than the width W. As explained below, H is minimized to facilitate insertion and positioning of implant 22 in the disk space from which all or a portion of the intervertebral disk 20 was removed and W is maximized to provide the desired stabilization to adjacent cervical vertebrae 16 and 18 (see Fig. 2). Third and fourth sides 34 are arched from one end of implant 22 to the other to provide the portion of implant 22 intermediate the ends 25 and 36 with a width W' larger than the width W" at the ends 25 and 36. Because the sides 32 of implant 22 are substantially flat and the sides 34 are arched from one end to the other, implant 22 is characterized as being a biplanar, bi-convex implant.

The bi-convex sides 32 of implant 22 are provided with teeth 38 for biting into adjacent vertebrae 16 and 18 as explained below. The end 36 of implant 22 is formed in a blunt, preferably flat, shape to reduce the likelihood of injury to the nerves during insertion into the disk space.

Implant 22 is provided with cleats 40 formed integrally therewith; the cleats 40 may be threaded onto or otherwise assembled to the implant 22 in a manner known in the art. Cleats 40 are diamond-shaped and oriented at an angle to implant 22 so that the portion of the cleat 40 which contacts the body of the adjacent vertebrae when rotated in the disk space as described below is a sharp, leading edge 42 to facilitate biting into the vertebrae 16 and 18. It will be recognized from this description that cleat 40 may be hooked, pointed, barbed, toothed, or formed in other shapes which accomplish the function of biting into the bone of the adjacent vertebrae to decrease the likelihood of anterior/posterior movement of the implant 22 (relative to the disk space) once inserted into the disk space. Similarly, teeth 38 need not be shaped as shown in Fig. 1 to decrease the likelihood of anterior-posteriormovement of implant 22 in the disk space. The teeth 38 can be pointed, beveled to a point in opposite directions, or otherwise shaped to increase the resistance to movement of implant 22 relative to the bone comprising the adjacent vertebrae 16 and 18.

Lock 24 is comprised of a stabilizer bar 29 detachably mounted to the end 25 of implant 22 which resists rotation of implant 22 relative to adjacent vertebrae 16 and 18 when implant 22 is inserted therebetween. As shown in Fig. 1, stabilizer bar 29 is mounted to implant 22 on a post 26 formed integrally with the end 25 of implant 22. As described below, the end 25 of implant 22 is positioned anteriorally when implant 22 is inserted between cervical vertebrae 16 and 18. Post 26 is threaded to receive a nut 30 for retaining the stabilizer bar 29 thereon. Means is provided for preventing rotation of stabilizer bar 29 relative to implant 22 when stabilizer bar 29 is mounted thereto in the form of the slots 46 formed on the end 25 of implant 22 for receiving a complementary-shaped key 48 formed on stabilizer bar 29; it will be recognized from this description that slot 46 may be located on implant 22 and key 48 may be located on stabilizer bar 29 without any difference in the manner in which these component parts function.

As best shown in Fig. 3, stabilizer bar 29 is elongate and is mounted to the end 25 of implant 22 so that the longitudinal axis of the stabilizer bar is positioned at an angle of about 90° relative to the longitudinal axis of implant 22. The ventral surfaces of the bodies of the adjacent vertebrae 16 and 18 are rounded when viewed along the axis of the spinal column as shown in Fig. 3, and to accommodate that rounded shape, stabilizer bar 29 is also curved. Stabilizer bar 29 is provided with an ear 44, preferably at or near both ends thereof, which projects into the disk space between vertebrae 16 and 18 when the implant 22 to which stabilizer bar 29 is mounted is positioned between the adjacent cervical vertebrae. Each of the ears 44 protrudes into the space between the bodies of the adjacent vertebrae 16 and 18 and is provided with vertebrae bearing surfaces 49 on the top and bottom surfaces thereof. When stabilizer bar 29 is mounted to implant 22, which is in turn held in place against anterior/posterior movement by the cleats 40 and teeth 38, the bearing surfaces 49 prevent relative rotation between vertebrae 16 and 18 and implant 22. In this manner, stabilizer bar 29 locks implant 22 in place between the vertebrae 16 and 18.

As noted above, the space around the implant 22 in the intervertebral space is preferably packed with bone chips (not shown) to facilitate fusion of the adjacent vertebrae, the implant 22 maintaining the proper spacing between vertebrae. As also noted above, it is important to prevent extrusion of the bone chips posteriorally into engagement with the spinal cord and/or spinal nerves. To prevent such extrusion, the cervical stabilizer of the present invention is provided with elongate spinal cord protectors 50, the long axes of which extend from implant 22 at approximately a 90° angle, e.g., orthogonally to the long axis of the implant 22 as shown in Fig. 3. The protectors 50 extend orthogonally from the first and second sides 32 of implant 22, i.e., laterally when the implant is implanted into the patient, within the disk space to close off the posterior aspect of the disk space. In this manner, the protectors 50 prevent, or at least reduce the likelihood of, extrusion of the bone chips packed into the disk space posteriorally into engagement with the spinal cord.

As discussed in more detail below, the surfaces of the bodies of the vertebrae 16 and 18 adjacent the disk space are slightly concave such that the disk space is shaped somewhat like the space between two inverted saucers. Due to the shape of the disk space, the posterior opening of the disk space narrows near the edges of the vertebral bodies, hence the proximal ends of protectors 50 are of smaller vertical dimension (vertical with reference to the patient's spinal column when the implant is implanted and the protectors 50 in the manner described below) at their distal ends than at the proximal ends 52 adjacent implant 22.

Again due to the shape of the disk space, it is inconvenientto insert implant 22 into the disk space with the protectors 50 in the position shown in Fig. 3, i.e., with the longitudinal axis of the protectors 50 orthogonal to the axis of implant 22. Means is therefore provided for positioning the protectors in that orthogonal position after implant 22 is inserted into the disk space. As shown in Fig. 1, the positioning means takes the form of snap fit posts 56 formed on each protector 50 for press-fitting into complementary slots 58 formed in the surface 60 of an end stop 62 integral with the end 36 of implant 22. Surface 60 is oriented at approximately 90° to the surfaces 32 of implant 22 and the posts 56 are formed on one of the sides 64 of protectors 50 so that when the posts 56 are press fit into the slots 58, the long axes of the protectors 50 are positioned at the same orthogonal angle (relative to the longitudinal axis of implant 22) as the surface 60.

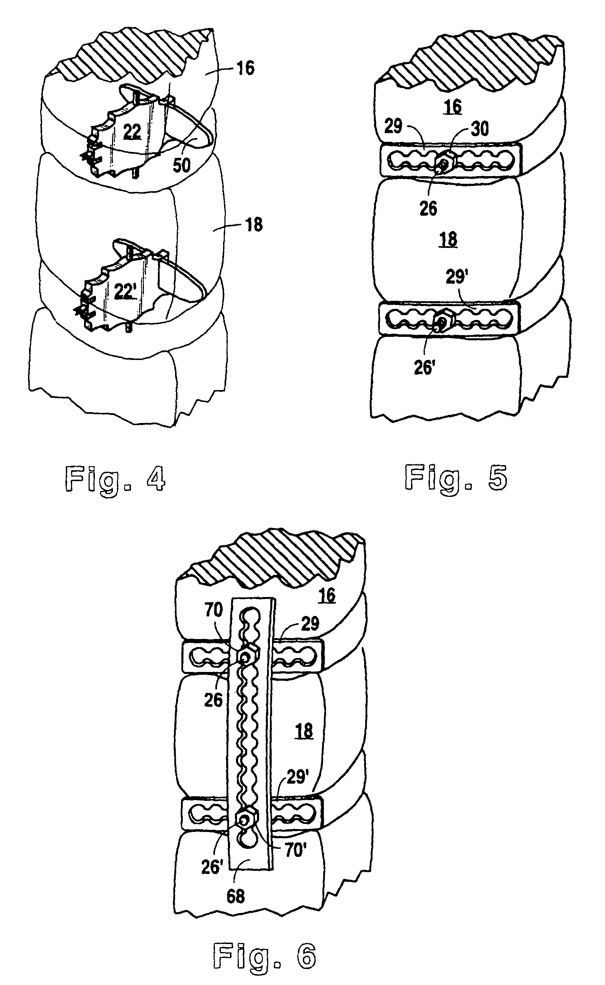

Referring now to Figures 4-6, a spinal stabilizer including the cervical stabilizer of the present invention is shown. The bodies of three adjacent cervical vertebrae 16, 18, and 66 are shown from the ventral side of the patient (the anterior, or front, of the disk space) in Fig. 4. A first cervical stabilizer 22 is inserted into the space between first and second vertebrae 16 and 18 and a second stabilizer 22' is inserted into the space between second and third vertebrae 18 and 66. As shown in Fig. 5, stabilizer bars 29 and 29' are mounted to each of the implants 22 and 22' on the posts 26 and 26' of each implant using nuts 30 and 30', respectively, as described above. As shown in Fig. 6, connector 68 is then fastened the posts 26 and 26' of implants 22 and 22' with nuts 70 and 70' to link the first and second implants 22 and 22', thereby supporting fusion of three cervical vertebrae with all the advantages of the cervical stabilizer of the present invention.

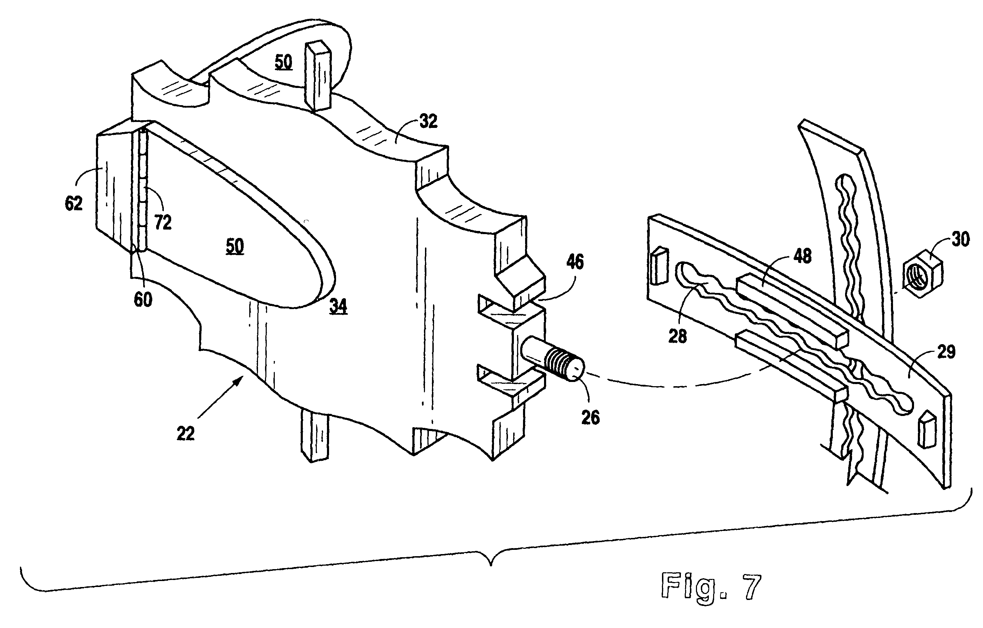

Figs. 7-12 show alternative embodiments of the cervical stabilizer of the present invention. Referring to Fig. 7, it can be seen why the connection between the protectors 50 and implant 22 is referred to as a positioning means. As shown in Figs. 1-6, the positioning means comprises press fit connectors formed by the posts 56 and slots 58 on protectors 50 and end stop 62, respectively. In Fig. 7, the positioning means takes the form of a piano hinge 72 connecting protectors 50 to the end stop 62. The implant 22 is inserted into the intervertebral space with the protectors 50 in a first, folded position with the longitudinal axes of protectors 50 substantially parallel to the longitudinal axis of the implant 22. After rotation of the implant 22 along the longitudinal axis thereof in the manner described above, the protectors 50 are pivoted from the first, folded position to the second, extended position in which the longitudinalaxes of the protectors are substantially orthogonal to implant 22. To illustrate, the implant 22 shown in Fig. 7 is shown with one protector 50 in the first, folded position and the other in the second, extended position.

As shown in Fig. 7, the piano hinge 72 is formed on the end stop 62 near the point at which the end stop 62 and the sides 34 of implant intersect. In this manner, the orthogonal surface 60 of end stop 62 acts to provide a stop to prevent the pivoting of the protectors 50 from the first position past the second position in which the protectors extend substantially perpendicularly from the longitudinal axis of implant 22 and helps retain the protector 50 in the second, extended position.

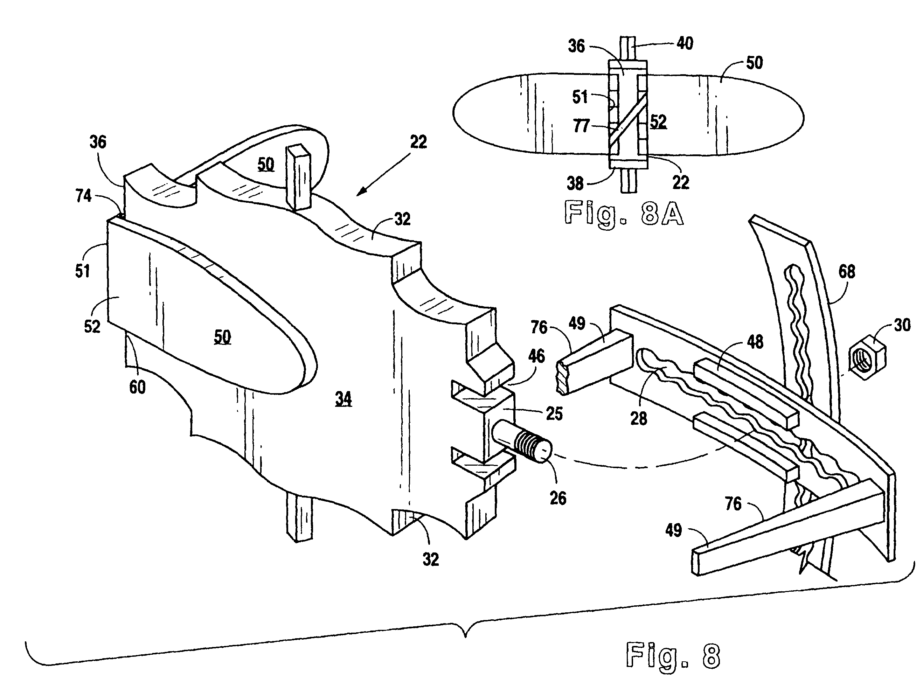

In a third embodiment shown in Fig. 8, the protectors 50 are pivoted from a first, folded position to the second, extended position on piano hinges 74. In this embodiment, the piano hinges 74 connecting protectors 50 to implant 22 are located on the end 36 of implant 22 so that, when the protectors 50 are pivoted to the extended position, the surfaces 51 at the proximal ends 52 abut so as to resist further pivoting of the protectors 50 past the second position. Note also that the ears 76 of the stabilizer bar 29 shown in Fig. 8 are shaped differently than the ears 44 shown in the embodiments shown in Figs. 1-7. Ears 76 extend from stabilizer bar 29 for a substantial distance so that, when the stabilizer bar 29 is mounted to implant 22, they project into the intervertebral space so that the vertebrae engaging surfaces 49 formed thereon engage the hard cortical bone at the edges of the bodies of the adjacent vertebrae 16 and 18, thereby providing support for the adjacent vertebrae in a position located outwardly from the implant 22.

Because their function is to contain the bone chips packed into the disk space on either side of the implant 22 (rather than to maintain the spacing between the adjacent vertebrae), the protectors 50 need not be comprised of the same material which comprises implant 22. Protectors 50 may, for instance, be comprised of a semi-rigid, biocompatible polymer. When comprised of such materials, the cervical stabilizer of the present invention can be made in a configuration,shown in Fig. 8A, which enables its removal from the disk space. Further, unlike known intervertebral implants, the cervical stabilizer of the present invention can be removed after fusion of the bone chips around the implant.

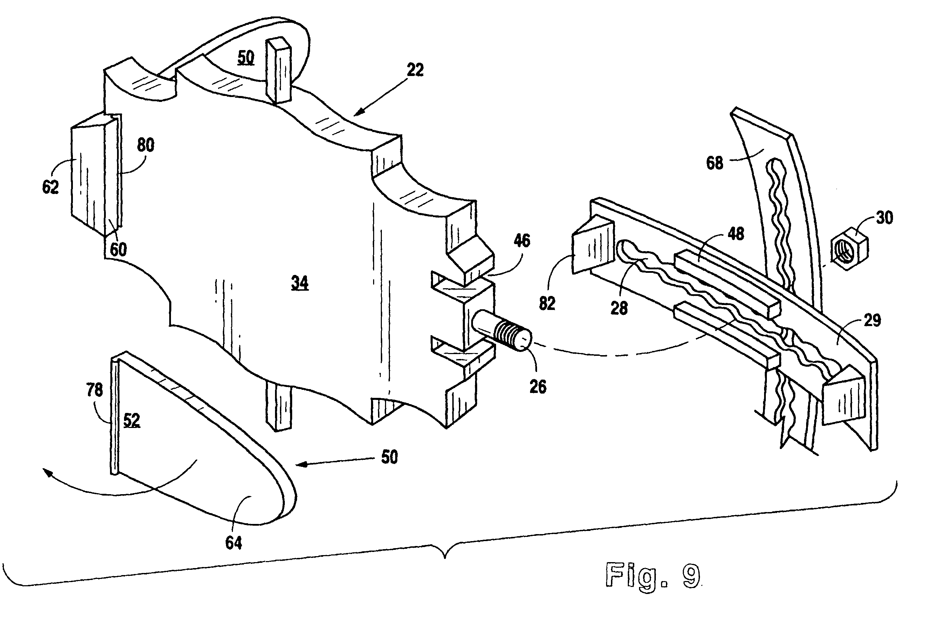

Removal is enabled by casting or otherwise fabricating the protectors 50 from a semi-rigid polymer with a spacer 77 as shown in Fig. 8A. As shown in an elevational view of the end 36 of implant 22 with the protectors 50 spread from the first, folded position to the second, extended position, protectors 50A and 50B are mounted on the comers of the end 36 of implant 22 rather than the end surface of end 36 as shown in Fig. 8. When mounted to the comers of the end 36 and pivoted to the second, extended position, a gap is left between the opposed back surfaces 51 of protectors 50A and 50B. However, as is the case in the embodiments shown in Figs. 7 and 8 (and Figs. 9-12 discussed below), the implant 22 shown in Figs. 8A is also provided with means for restraining the pivoting of the protectors 50 from the first, folded position past the second, extended position. As shown in Fig. 8A, the proximal end 52 of one of the protectors (identified as 50A in Fig. 8A) is provided with an integral spacer 77 which extends at an angle from the surface 51 to abut the opposed surface 51 of the proximal end 52 of protector 50B to resist pivoting of the protectors 50A and 50B past the second, extended position (i.e., to prevent the protectors from pivoting past an angle which is approximately 90° relative to the longitudinal axis of implant 22). If it is desired to remove the implant shown in Fig. 8A, the stabilizer bar 29 is removed from the end 25 of implant 22 and the implant 22 is grasped at the end 25 and pulled anteriorally in the disk space (ventrally to the patient). As the implant 22 moves anteriorally in the disk space, the protectors 50A and 50B are rotated past the orthogonal position described above, causing the spacer 77, comprised of the same semi-rigid material as protectors 50A and 50B, to be collapsed between the opposed surfaces 51, thereby allowing the protectors to effectively pivot to a third position in which they are once again substantially parallel to the longitudinal axis of implant 22 but extend in the opposite direction along that axis from the first, folded position. No pieces are broken off from the protectors or implant, nor does any part of the implant or protector stay behind in the space which is left by removal of the stabilizer.

Another embodiment of the cervical stabilizer of the present invention is shown in Fig. 9. In this embodiment, the protectors 50 are provided with a rolled edge 78 at the proximal end 52 thereof which is press fit into a complementary-shaped groove 80 formed in the orthogonal surface 60 of end stop 62. The orthogonal surface 60 provides both a stop against which the protector is positioned when inserted into the disk space for press fitting into the groove 80 and resistance to pivoting of the protector to an angle beyond about 90° from the longitudinal axis of implant 22 in the manner described in connection with the embodiments shown in Figs. 1-8. Also, the ears 82 of the embodiment shown in Fig. 9 are shaped and sized to protrude further into the disk space than the ears 44 of the embodiments shown in Figs. 1-7 to provide additional vertebral surface against which the vertebral bearing surfaces 49 bear.

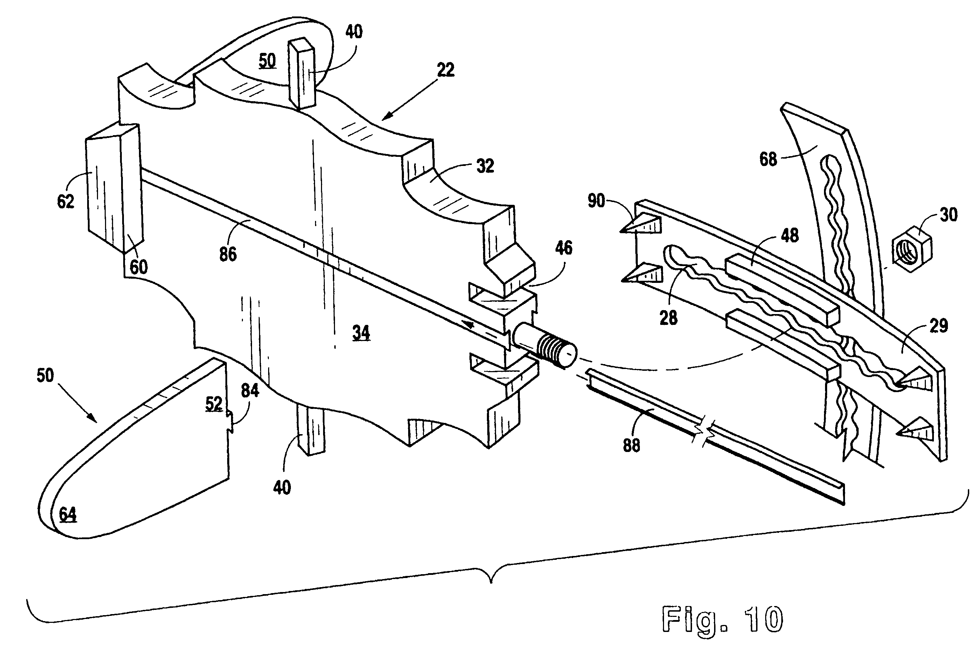

In the embodiment shown in Fig. 10, as in the embodiments of Figs. 1-6 and 9, protectors 50 are assembled to implant 22 after implant 22 is inserted into the intervertebral space. In the embodiment shown in Fig. 10, the proximal ends 52 of protectors 50 are provided with a key 84 for sliding in a keyway 86 running longitudinallyalong the length of the third and fourth faces 34 of implant 22 until protectors 50 abut the orthogonal surface 60 of end stop 62, which comprises the means for resisting the pivoting of the protectors 50 past the second, extended position as described above. To prevent the protectors 50 from working out of the keyway 86 toward the end 25 of implant 22, an elongate keeper 88, shaped to fit into and slide in keyway 86, is provided so that when the keeper 88 is inserted into the keyway 84 and the stabilizer bar 29 is mounted thereto, the keeper 88 prevents movement of the protectors 50.

In the embodiment shown in Fig. 10, the vertebral bearing surfaces 49 on the stabilizer bar take the form of multiple, pointed barbs 90. If there is insufficient space between adjacent vertebrae for the ears 44 of the stabilizer bars 29 shown in Figs. 1-9 to fit between the bodies of the vertebrae, or if for some other reason a stabilizer bar having ears formed thereon cannot be utilized, relative rotational movement between vertebrae and implant can be resisted by tightening the nut 30 to the point that the barbs 90 are driven into the surfaces of the bodies of the adjacent vertebrae.

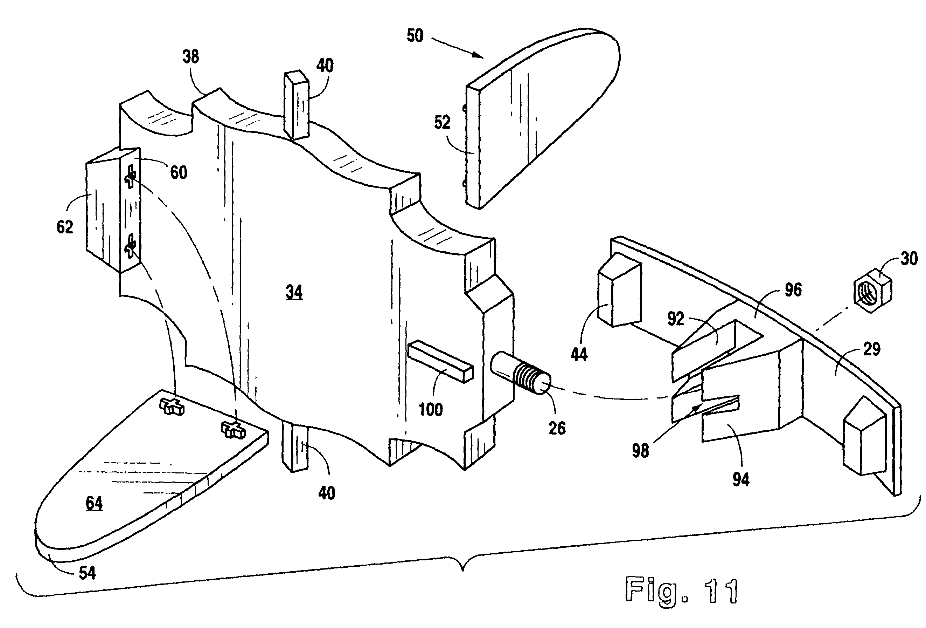

In the embodiment shown in Fig. 11, relative rotation between stabilizer bar 29 and implant 22 is prevented by interaction of the inside surfaces 92 of the arms 94 of the U-shaped lock 96 formed integrally with stabilizer bar 29 and the surfaces 34 of implant 22. The surfaces 92 of lock 96 are provided with a slot 98 for receiving a complementary-shaped key 100 on implant 22 to facilitate assembly of lock 96 to implant 22. It will be apparent from this description that slot 98 may be located on implant 22 and key 100 may be located on lock 96 without any difference in the manner in which these parts function. The lock 96 is provided with a bore (not shown) for receiving post 26 when the slot 98 is aligned with the key 100 on implant 22. In this embodiment, vertebrae bearing surfaces are provided on the ears 44 and on the top and bottom of lock 96.

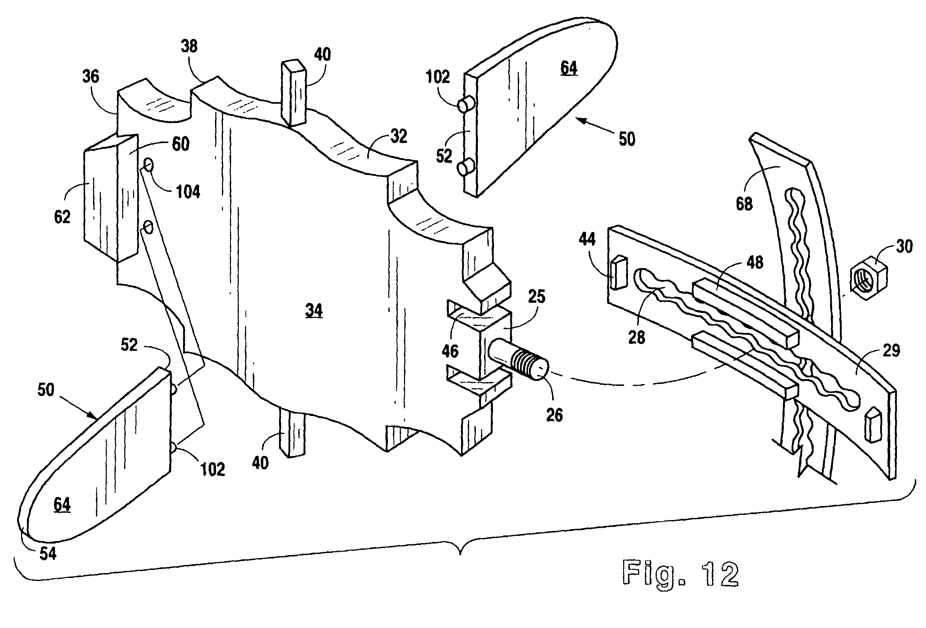

In the embodiment shown in Fig. 12, the protectors 50 are provided with posts 102 similar to the posts 56 of the embodiment shown in Figs. 1-6 for press-fitting into complementary-shaped slots 104 formed in the third and fourth surfaces 34 of implant 22. When the posts 102 are snapped into slots 104, the orthogonal surface 60 of end stop 62 abuts the sides, or flat surfaces, of protectors 50, thereby resisting pivotal movement of the protectors 50 relative to implant 22.

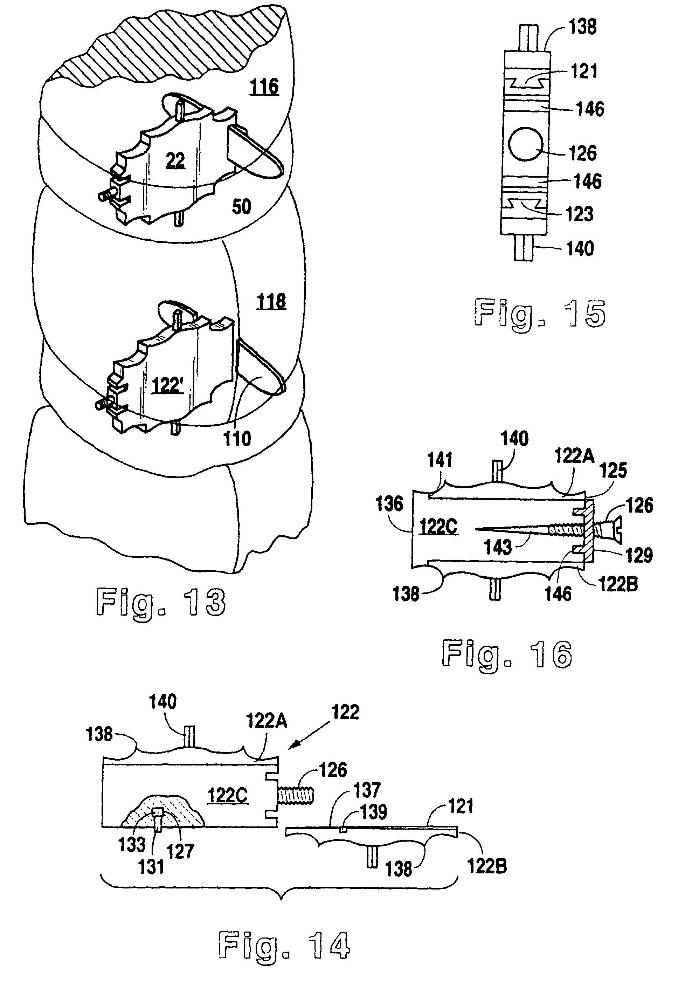

Another embodiment of the cervical stabilizer of the present invention is shown in place between two adjacent cervical vertebrae 116 and 118 in Fig. 13. Like the embodiment shown in Fig. 8A, the embodiment shown in Fig. 13 is capable of being removed from the disk space even after fusion of the bone chips packed into the disk space around the implant 122. As shown in Fig. 13, the protectors 110, rather than being hinged to the implant 122 and comprised of metal or the semi-rigid polymeric material described in connection with the embodiment shown in Fig. 8A, are themselves cut and shaped from bone so as to fit into and close off the posterior opening of the disk space (the dorsal aspect of the vertebrae). The pieces of bone 110 shown in Fig. 13 are effectively shaped by the surgeon during the surgery so that they can wedge between the adjacent vertebrae 116 and 118 during the after rotating the implant 122 as described above and before packing the disk space with bone chips so as to close off the posterior (dorsal) opening of the disk space. In the event the implant 122 needs to be removed at a later date such that the bone chips have fused, the bone protectors 110 will have fused with the bone chips and the implant 122 is simply removed without the protectors 110.

In the embodiments shown in Figs. 14 and 15, the component parts of implant 122 corresponding to like parts of the various implants 22 shown in Figs. 1-12 are referenced by the same reference numerals preceded by a "1", i.e. the teeth 38 are shown at 138 in Figs. 14-15. Implant 122 is constructed to facilitate removal of the cervical stabilizer of the present invention after fusion of the bone chips in the disk space. As shown in Fig. 14, implant 122 is comprised of sections 122A, 122B, and 122C divided along the longitudinal axis of implant 122. Sections 122A, 122B, and 122C are interconnected by interlocking tongues 121 and grooves 123, the tongues 121 being formed on the sections 122A and 122B and the grooves 123 being formed on section 122C (Fig. 15). Section 122C is provided with means for selectively preventing relative movement of the sections 122A, 122B, and 122C in the form of a pair of blind pockets 127 (only one of which is shown in Fig. 14) having pins 131 positioned therein, each pin 131 being biased outwardly by a spring 133. The tongues 121 near each of the ends of sections 122A and 122B which, when assembled to section 122C, comprise the end 136 of implant 132 are inclined as at 137 so that the pins 131 ride up along the incline 137 as each section 122A and 122B slides onto implant section 122C to pop into a detent 139 formed in the surface of the tongue 121 to lock the sections 122A and 122B in place on section 122C.

The detents 139 formed in the surfaces of the tongues 121 of sections 122A and 122B are relatively shallow so that, when sufficient force is exerted on the section 122C in a direction along the longitudinal axis of implant 122 such as when the implant 122 is to be removed from the disk space, the pins 131 are unable to remain in the position in which they are biased into the detents 139. Once the implant section 122C has been removed from between sections 122A and 122B in this fashion, the sections 122A and 122B are then dissected free from the fused bone and withdrawn from the opening left by removal of section 122C. In an alternative embodiment, rather than making the detents in sections 122A and 122B shallow, the pins 131 are comprised of a material which breaks when subjected to lateral force, allowing the section 122C to be pulled from between sections 122A and 122B.

Figure 16 shows another three piece implant 122 intended to facilitate removal from the disk space. Rather than pins and detents as described in connection with the embodiment shown in Figs. 14 and 15, the implant 122 shown in Fig. 16 is provided with a center section 122C having a stop 141 formed near the end 136 thereof so that, when the sections 122A and 122B are slid onto the section 122C, their travel toward end 136 is restrained by the stops 141. To selectively prevent movement of sections 122A and 122B in a direction along the longitudinal axis of implant 122 in a direction away from the stops 141, the section 122C is provided with a threaded split 143 for receiving a screw 126 therein. When the screw 126 is inserted through the stabilizer bar 129 into the split 143 and tightened, the screw 126 forces the two halves of section 122C at the end 125 thereof apart. The slots 146 formed in the end 125 of implant 122 for receiving the keys 148 on stabilizer bar 129 are enlarged to allow relative movement between the two halves of section 122C and stabilizer bar 129. Because the sections 122A and 122B are rigidified by the shape of the tongues 121, sections 122A and 122B do not bend as the halves of section 122C are spread by screw 126, causing the sections 122A and 122B to bind with section 122C to prevent relative movement therebetween. To remove the implant 122 shown in Fig. 16 from the disk space, the screw 126 is loosened to allow the halves of section 122C to relax to their original shape, thereby allowing section 122C to move relative to sections 122A and 122B so that the section 122C can be removed from therebetween.

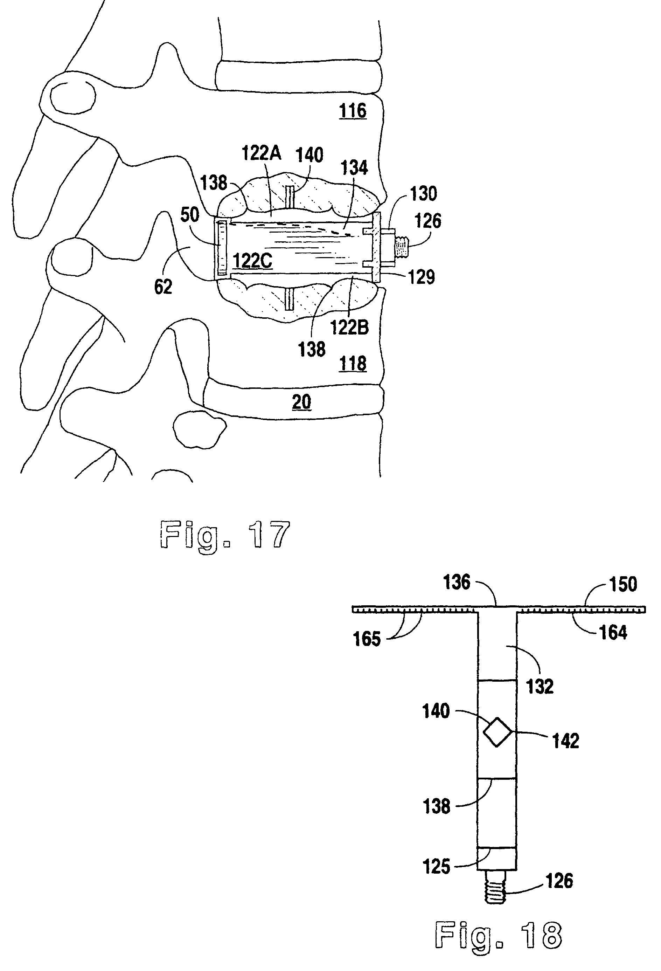

Yet another embodiment of a removable cervical stabilizer is shown implanted between two adjacent cervical vertebrae 116 and 118 in Fig. 17. In this embodiment, the implant 122 is again comprised of three sections 122A, 122B, and 122C and section 122C is provided with stops 141 near the end 136 thereof. However, in the embodiment shown in Fig. 17, relative movement of section 122C along the longitudinal axis of implant 122 away from stops 141 is selectively prevented by the stabilizer bar 129 which, when retained on the post 126 by nut 130, traps the sections 122A and 122B against the stops 141. By comparison of Figs. 2 and 17, it can be seen that the stabilizer bar 129 is of greater vertical (with reference to the patient's spinal column) dimension than the stabilizer bar 29 shown in Fig. 2. As shown in Fig. 17, bone protectors 110 are provided to prevent posterior (dorsal) extrusion of the bone chips packed into the disk space, but it will be recognized from this description that the implant 122 shown in Fig. 17 (or any of the implants shown in Figs. 14-16) can also be provided with protectors of the type described above in connection with Fig. 8A or as described below in connection with Fig. 18.

The implant 122 shown in Fig. 18 includes integrally formed protectors 150 which. like the protectors 50A and 50B described above in connection with Fig. 8A, take advantage of the fact that the protectors need not be as rigid and strong as the implant. As shown in Fig. 18, the protectors 150 are formed from the same material as implant 122 but are relatively thin. When force is exerted along the longitudinal axis of implant 122 to remove the implant 122 from the disk space, the thin protectors 150 bend at their respective junctions with the implant 122 to allow the implant and protectors 150 to be pulled from the disk space. If necessary, the surface 164 of protectors 150 is provided with a plurality of vertical, regularly spaced grooves, or scoring, as shown at 165 to allow the protectors 150 to continue to bend so as to "feed" through the slot in the fused bone around the implant 122 as implant 122 is pulled from between the cervical vertebrae.

Referring again to Figs. 1-7, the method of the present invention will be described. Using spreaders as disclosed in International Application No. PCT/US95/00347, the vertebrae 16 and 18 are distracted to open the disk space. When the desired "spread" is achieved, an implant 22 having a height H and width W selected to fit the disk space is mounted on an applicator (not shown) by screwing the implant 22 onto threads formed on the end of the applicator on the threads on post 26. The implant 22 is then inserted into the disk space using the applicator 52 with the implant 22 oriented so that the top and bottom thereof, i.e., the first and second sides 32, engage the bodies of the adjacent vertebrae 16 and 18. Using the applicator, the implant 22 is then moved further into (or out of) the disk space in an anterior-posterior direction to position implant 22 in the disk space at a position in which the expanded, middle portion and the smaller ends 25 and 36 of the third and fourth sides 34 of implant 22 contact the respective lower and upper surfaces of the bodies of the vertebrae 16 and 18 when rotated approximately 90° using the applicator. The respective lower and upper surfaces of the vertebral bodies are slightly concave such that the larger width middle portion W" of implant 22 allows the implant 22 to engage substantially more of the respective surfaces of the vertebral bodies than conventional implants, thereby providing increased stability to the fusion once further rotation of implant 22 in the disk space is prevented as described below.

When positioned to provide maximum stabilization and rotated by about 90° so that the surfaces 32 of implant 22 contact the bodies of vertebrae 16 and 18, the applicator is unscrewed from implant 22 and backed out of the disk space. The protectors 50 are then inserted into the disk space and press fit into place in the slots 58 formed in the orthogonal surface 60 of end stop 62. In the case of the alternative embodiments in which the means for positioning the protectors 50 comprises hinges or other such structure, the protectors 50 are pivoted from the first position in which the axis of the protector is substantially parallel to the axis of implant 22 to the second position in which the axis of the protector is orthogonal to the axis of the implant. The space within the disk space on either side of the implant 22 is then packed with cancellous bone chips.

After packing the disk space with bone chips, the stabilizer bar 29 is inserted through the same incision with the ears 44 protruding into the disk space from the anterior aspect of the disk space (the patient's ventral side). Nut 30 is then threaded onto post 26 and tightened to mount the stabilizer bar 29 to implant 22 and hold the ears 44 in the disk space. Securing the stabilizer bar 29 to implant 22 in this manner prevents relative rotation between stabilizer bar 29 and implant 22 while the ears 44 of stabilizerbar 29 protrude into the disk space between the bodies of the adjacent vertebrae 16 and 18 so that the bearing surfaces 49 prevent rotation of the stabilizer bar 29 relative to the vertebrae 16 and 18. The vertebrae bearing surfaces 49 bear against the cortical end plates, which are comprised of non-cancellous bone, of the respective vertebral bodies which provide a hard, relatively smooth surface against which the surfaces 49 bear.

If necessary, a small amount of a physiologically compatible adhesive of a type known in the art is applied over the cancellous bone chips in the disk space to close off the opening into the disk space; the bone chips start fusing before the opening narrows. The patient should be able to sit and ambulate soon after the procedure is completed because of the stability of the implant. Removal of the implant, if necessary, has been described above in connection with the description of Figs. 8A and 13-18.

Although described in terms of the embodiments shown in the figures, these embodiments are shown to exemplify the invention, it being apparent that changes can be made to the embodiments shown without departing from the spirit of the present invention. Such changes may include, for instance, forming the implant 22 so that the first and second sides are substantially flat but not parallel along their longitudinal axes. The resulting wedge shape of the implant facilitates insertion of the implant into the disk space, the rounded end reducing the likelihood of injury to the nerves of the spinal cord during insertion into the disk space. Likewise, several combinations of positioning means, rotation preventing means, and ear shapes are described herein and it will be recognized that these combinations can be utilized in any of several different combinations with equal efficacy.

## Claims

1. A cervical disk stabiliser for insertion between originally adjacent vertebrae, the stabiliser comprising an elongate implant (22) having a substantially rectangular cross-sectional shape for inserting between originally adjacent cervical vertebrae; a stabiliser bar (29) for detachable mounting to one end of said implant and means (46, 48) to prevent relative rotation between the stabiliser bar and the implant when the stabiliser bar is mounted on the implant, there being ears (44) formed on the stabiliser bar for insertion into the disc space between said adjacent vertebrae, said ears (44) having surfaces for bearing against the adjacent cervical vertebrae, to prevent movement of said stabiliser bar (29) relative to the adjacent cervical vertebrae.

2. The stabiliser of Claim 1 wherein the stabiliser further comprises a spinal column protector (50) for mounting to the end of the implant (22) opposite the said one end.

3. The stabiliser of Claim 2 wherein said protector (50) extends outwardly from said implant at an angle of about 90° relative to the longitudinal axis of said implant (22).

4. The stabiliser of Claims 2 or 3 wherein said protector (50) is pivotally mounted to said implant (22).

5. The stabiliser of Claim 4 wherein said protector (50) is adapted to pivot from a first, folded position against said implant (22) to a second, extended position extending orthogonally from said implant (22).

6. The stabiliser of Claim 4 wherein said protector (50) is adapted to be pivoted from a first, folded position for inserting said implant (22) into the space between adjacent cervical vertebrae, and second position extending outwardly from said implant at an angle relative to the longitudinal axis of said implant (22) for closing off the dorsal aspect of the space between adjacent cervical vertebrae.

7. The stabiliser of any one of Claims 5 or 6 additionally comprising means (60) for restraining the pivoting of said protector (50) from the first, folded position past said second, extended position.

8. The stabiliser of Claim 7 wherein said restraining means (77) is breakable so as to allow said protector (50) to pivot past said second, extended position to a third position approximately 180° from said first, folded position.

9. The stabiliser of any one of Claims 2 to 5 wherein said protector (150) is provided with a plurality of vertically aligned grooves (165) for allowing said protector to bend.

10. The stabiliser of any one of the preceding Claims wherein the longitudinal axis of said stabiliser bar (29) is positioned at an angle of about 90° to the longitudinal axis of said implant (22).

11. The stabiliser of any one of the preceding Claims wherein stabiliser bar (29) is curved to approximate the shape of ventral aspect of a vertebra.

12. The stabiliser of any one of the preceding Claims additionally comprising a connector (24) for detachably mounting to said implant (22) when implanted between adjacent cervical vertebrae for connecting the implant to an adjacent implant.

13. The stabiliser of any one of the preceding Claims wherein the implant (22) is comprised of sections (112A, 122B, 122C) that are movable with respect to each other.

## Patentansprüche

1. Zervikalbandscheibenstabilisator zum Einsetzen zwischen ursprünglich angrenzenden Wirbeln, wobei der Stabilisator ein längliches Implantat (22) mit einer im wesentlichen rechtwinkligen Querschnittsform zum Einsetzen zwischen ursprünglich angrenzenden Zervikalwirbeln; einen Stabilisatorstab (29) zum abnehmbaren Montieren an einem Ende des Implantats und Mittel (46, 48), um eine Relativdrehung zwischen dem Stabilisatorstab und dem Implantat zu verhindern, wenn der Stabilisatorstab an dem Implantat montiert ist, umfaßt, wobei es Zipfel (44) gibt, die an dem Stabilisatorstab zum Einsetzen in den Bandscheibenraum zwischen den angrenzenden Wirbeln gebildet sind, wobei die Zipfel (44) Oberflächen zum Stützen gegen die angrenzenden Zervikalwirbel aufweisen, um eine Bewegung des Stabilisatorstabs (29) relativ zu den angrenzenden Zervikalwirbeln zu verhindern.

2. Stabilisator nach Anspruch 1, **dadurch gekennzeichnet, daß** der Stabilisator ferner eine Wirbelsäulenschutzeinrichtung (50) zum Montieren an dem Ende des Implantats (22) gegenüberliegend dem besagten einen Ende umfaßt.

3. Stabilisator nach Anspruch 2, **dadurch gekennzeichnet, daß** die Schutzeinrichtung (50) sich nach außen von dem Implantat mit einem Winkel von etwa 90° relativ zu der Längsachse des Implantats (22) erstreckt.

4. Stabilisator nach Anspruch 2 oder 3, **dadurch gekennzeichnet, daß** die Schutzeinrichtung (50) schwenkbar an dem Implantat (22) montiert ist.

5. Stabilisator nach Anspruch 4, **dadurch gekennzeichnet, daß** die Schutzeinrichtung (50) angepaßt ist, um sich von einer ersten, gefalteten Position gegenüber dem Implantat (22) zu einer zweiten, ausgeweiteten Position zu verschwenken, die sich orthogonal von dem Implantat (22) erstreckt.

6. Stabilisator nach Anspruch 4, **dadurch gekennzeichnet, daß** die Schutzeinrichtung (50) angepaßt ist, um sich von einer ersten, gefalteten Position zum Einsetzen des Implantats (22) in den Raum zwischen benachbarten Zervikalwirbeln und einer zweiten Position zu verschwenken, die sich von dem Implantat mit einem Winkel relativ zu der Längsachse des Implantats (22) nach außen zum Absperren der Rückenseite des Raums zwischen angrenzenden Zervikalwirbeln erstreckt.

7. Stabilisator nach einem der Ansprüche 5 oder 6, welcher zusätzlich Mittel (60) zum Einschränken des Verschwenkens der Schutzeinrichtung (50) aus der ersten, gefalteten Position hinter die zweite, ausgeweitete Position umfaßt.

8. Stabilisator nach Anspruch 7, **dadurch gekennzeichnet, daß** das Einschränkungsmittel (77) zerbrechlich ist, um es der Schutzeinrichtung (50) zu ermöglichen, sich hinter die zweite, ausgeweitete Position zu einer dritten Position, etwa 180° von der ersten, gefalteten Position, zu verschwenken.

9. Stabilisator nach einem der Ansprüche 2 bis 5, **dadurch gekennzeichnet, daß** die Schutzeinrichtung (150) mit einer Vielzahl von vertikal ausgerichteten Rillen (165) bereitgestellt ist, damit die Schutzeinrichtung sich biegen kann.

10. Stabilisator nach einem der vorangehenden Ansprüche, **dadurch gekennzeichnet, daß** die Längsachse des Stabilisatorstabs (29) mit einem Winkel von etwa 90° zu der Längsachse des Implantats (22) positioniert ist.

11. Stabilisator nach einem der vorangehenden Ansprüche, **dadurch gekennzeichnet, daß** der Stabilisatorstab (29) zu ungefähr der Form der ventralen Seite eines Wirbels gekrümmt ist.

12. Stabilisator nach einem der vorangehenden Ansprüche, welcher zusätzlich eine Verbindungseinrichtung (24) zum abnehmbaren Montieren an das Implantat (22), wenn es zwischen angrenzenden Zervikalwirbeln implantiert ist, zum Verbinden des Implantats an ein angrenzendes Implantat umfaßt.

13. Stabilisator nach einem der vorangehenden Ansprüche, **dadurch gekennzeichnet, daß** das Implantat (22) von Abschnitten (112A, 122B, 122C) umfaßt ist, die in Bezug zueinander beweglich sind.

## Revendications

1. Un stabilisateur de disque cervical, pour insertion entre des vertèbres à l'origine adjacentes, le stabilisateur comprenant un implant (22) allongé, ayant une forme en section transversale sensiblement rectangulaire, pour insertion entre des vertèbres cervicales à l'origine adjacentes ; une barre stabilisatrice (29) pour montage désolidarisable sur une première extrémité dudit implant, et des moyens (46,48) pour empêcher toute rotation relative entre la barre stabilisatrice et l'implant lorsque la barre stabilisatrice est montée sur l'implant, des oreilles (44) étant formées sur la barre stabilisatrice, pour insertion dans l'espace de disque entre lesdites vertèbres adjacentes, lesdites oreilles (44) comprenant des surfaces devant porter contre les vertèbres cervicales adjacentes, pour empêcher tout déplacement de ladite barre stabilisatrice (29) par rapport aux vertèbres cervicales adjacentes.

2. Stabilisateur selon la revendication 1, dans lequel le stabilisateur comprend en outre un protecteur de colonne spinale (50), devant être monté sur l'extrémité de l'implant (22) opposée à ladite première extrémité.

3. Stabilisateur selon la revendication 2, dans lequel ledit protecteur (50) s'étend à l'extérieur dudit implant, sous un angle d'environ 90° par rapport à l'axe longitudinal dudit implant (22).

4. Stabilisateur selon la revendication 2 ou 3, dans lequel ledit protecteur (50) est monté à pivotement sur ledit implant (22).

5. Stabilisateur selon la revendication 4, dans lequel ledit protecteur (50) est adapté pour pivoter, depuis une première position, repliée contre ledit implant (22), à une deuxième position, déployée, s'étendant à angle droit par rapport audit implant (22).

6. Stabilisateur selon la revendication 4, dans lequel ledit protecteur (50) est adapté pour être pivoté, d'une première position repliée, pour insertion dudit implant (22) dans l'espace se trouvant entre les vertèbres cervicales adjacentes, à une deuxième position s'étendant à l'extérieur dudit implant sous un certain angle par rapport à l'axe longitudinal dudit implant (22), afin de fermer la surface dorsale de l'espace se trouvant entre des vertèbres cervicales adjacentes.

7. Stabilisateur selon l'une quelconque des revendications 5 ou 6, comprenant en plus des moyens (60) pour limiter le pivotement dudit protecteur (50), partant de ladite première position repliée et tentant de franchir ladite deuxième position déployée.

8. Stabilisateur selon la revendication 7, dans lequel lesdits moyens de retenue (77) sont fracturables, pour permettre audit protecteur (50) de pivoter plus amplement qu'à ladite deuxième position déployée, en passant à une troisième position, orientée à peu près à 180° de ladite première position repliée.

9. Stabilisateur selon l'une quelconque des revendications 2 à 5, dans lequel ledit protecteur (150) est muni d'une pluralité de gorges (165) alignées verticalement, devant permettre la flexion dudit protecteur.

10. Stabilisateur selon l'une quelconque des revendications précédentes, dans lequel l'axe longitudinal de ladite barre stabilisatrice (29) est orientée sous un angle d'environ 90° par rapport à l'axe longitudinal dudit implant (22).

11. Stabilisateur selon l'une quelconque des revendications précédentes, dans lequel ladite barre stabilisatrice (29) est incurvée, pour adopter à peu près la forme de la surface ventrale d'une vertèbre.

12. Stabilisateur selon l'une quelconque des revendications précédentes, comprenant en plus un connecteur (24), servant au montage désolidarisable sur ledit implant (22) lorsqu'il est implanté entre des vertèbres cervicales adjacentes, afin de connecter l'implant à un implant adjacent.

13. Stabilisateur selon l'une quelconque des revendications précédentes, dans lequel l'implant (22) est formé de sections (112A, 122B, 122C) déplaçables les unes par rapport aux autres.
